(19) Europäisches Patentamt
European Patent Office
Office européen des brevets

(11) Numéro de publication: **0 208 621 B1**

(12) **FASCICULE DE BREVET EUROPEEN**

(45) Date de publication du fascicule du brevet: **12.04.89**

(51) Int. Cl.⁴: **C 07 D 471/04,** A 61 K 31/435 // (C07D471/04, 221:00, 221:00)

(21) Numéro de dépôt: **86401537.5**

(22) Date de dépôt: **10.07.86**

(54) **Amides substitués, leur préparation et compositions qui les contiennent.**

(30) Priorité: **11.07.85 FR 8510619**
**16.01.86 FR 8600555**

(43) Date de publication de la demande:
**14.01.87 Bulletin 87/3**

(45) Mention de la délivrance du brevet:
**12.04.89 Bulletin 89/15**

(84) Etats contractants désignés:
**AT BE CH DE FR GB IT LI LU NL SE**

(56) Documents cités:
**EP-A- 0 172 083**
**FR-A- 1 006 725**

(73) Titulaire: **RHONE-POULENC SANTE, 20, avenue Raymond Aron, F-92160 Antony (FR)**

(72) Inventeur: **Cotrel, Claude, 17 A, avenue du Docteur Arnold Netter, F-75012 Paris (FR)**
Inventeur: **Guyon, Claude, 17 bis, avenue Henri Martin, F-94100 St-Maur-des-Fossés (FR)**
Inventeur: **Roussel, Gérard, 20 ter, rue des Carrières, F-91450 Soisy-sur-Seine (FR)**
Inventeur: **Taurand, Gérard, 13, Passage Saillenfait, F-94000 Créteil (FR)**

(74) Mandataire: **Savina, Jacques et al, RHONE-POULENC INTERSERVICES Service Brevets Pharma 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

Dans la demande de brevet PCT 8 400 489 ont été décrits des dérivés de benzamides de formule générale:

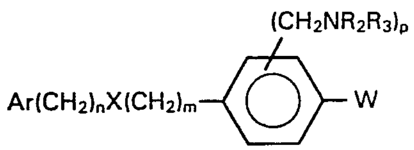

dans laquelle X représente notamment $-NR_1CO-$, qui sont utiles comme antiarythmiques.

Dans la demande de brevet néerlandais 7 305 482 et le brevet américain 3 993 656 ont été décrits des dérivés de naphtyridines-1,8 de structure:

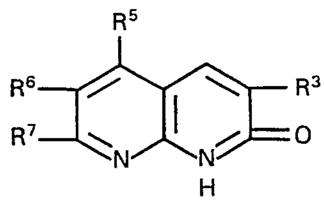

dans laquelle $R^6$ est entre autres un radical -NH-alcanoyle. Ces produits sont utiles comme bronchodilatateurs, vasodilatateurs périphériques et anti-hypertenseurs.

La présente invention concerne de nouveaux amides substitués répondant à la formule générale:

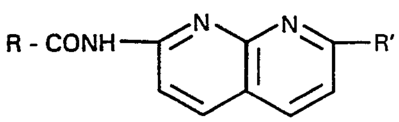

(I)

leur préparation et les médicaments qui les contiennent.

Dans la formule générale (I),

1) soit le symbole R représente un radical quinolyle-2, phényle substitué (par un atome de brome, par un atome de chlore en position -3, un radical trifluorométhyle ou alcoyloxycarbonyle ou par un radical dialcoylamino en position 4) ou un radical dichlorophényle, trialcoyloxyphényle, benzyle, styryle, benzoyle ou anilino, et

le symbole R' représente un atome d'halogène, un radical phényle ou anilino, ou bien

le symbole R' représente un radical phénoxy ou chloro-4 phénoxy lorsque R est un radical quinolyle-2, phényle substitué (par un atome de brome ou en position -3 par un atome de chlore ou substitué par un radical trifluorométhyle ou alcoyloxycarbonyle ou en position -4 par un radical dialcoylamino) ou trialcoyloxyphényle, benzyle ou styryle, ou bien

le symbole R' représente un radical fluoro-4 phényle ou alcoyloxy-4 phényle lorsque R est un radical trifluorométhyl-4 phényle ou dialcoylamino-4 phényle, ou bien

le symbole R' représente un radical bromo-3 phényle lorsque R est un radical anilino,

2) soit le symbole R représente un radical phényle ou alcoyloxycarbonyle et le symbole R' représente un radical phényle, chloro-4 phénoxy, anilino ou pyrrolidinyle-1, ou bien

le symbole R' représente un radical chloro-2 (ou -4) phényle, bromo-3 (ou -4) phényle, alcoyloxy-4 phényle ou dichloro (ou dibromo) -3, 4 phényle lorsque R est un radical phényle,

3) soit le symbole R représente un radical phényle substitué en position -3 par un atome d'halogène et

le symbole R' représente un radical fluoro-3 (ou -4) phényle, brome-3 (ou -4) phényle, chlore-4 phényle, alcoyloxy-2 (ou -3) phényle, alcoyl-3 phényle, ou difluoro (dichloro ou dibromo) -3, 4 phényle,

étant entendu que les radicaux alcoyle cités ci-dessus sont droits ou ramifiés et contiennent 1 à 4 atomes de carbone.

Selon l'invention, les produits de formule générale (I) dans laquelle R est défini comme précédemment à l'exception de représenter un radical anilino, et R' a la définition correpondante, peuvent être préparés par action d'un acide de formule générale:

$$R\text{-}COOH \qquad (II)$$

dans laquelle R est défini comme précédemment, ou d'un dérivé réactif de cet acide, sur une amine de formule générale:

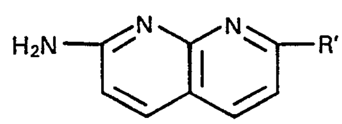

(III)

dans laquelle R' est défini comme précédemment.

Lorsque l'on met en oeuvre l'acide de formule générale (II), on opère en présence d'un agent de condensation peptidique tel qu'un carbodiimide (par exemple le dicyclohexylcarbodiimide) ou le N,N'-carbonyldiimidazole ou l'éthoxy-2 éthoxycarbonyl-1 dihydro-1, 2 quinoléine, dans un solvant organique tel qu'un éther (par exemple tétrahydrofuranne, dioxanne, glyme, diglyme), un amide (par exemple diméthylformamide), un nitrile (par exemple acétonitrile), un solvant chloré (par exemple chlorure de méthylène, dichloréthane ou chloroforme) ou un hydrocarbure aromatique (par exemple toluène), à une température comprise entre 0° C et la température de reflux du mélange réactionnel. De préférence on opère à 20°C.

Lorsque l'on met en oeuvre un dérivé réactif de l'acide de formule générale (II) il est possible de faire réagir l'anhydride, un anhydride mixte, un halogénure d'acide ou un ester [qui peut être choisi parmi les esters activés ou non de l'acide de formule générale (II)]. On opère alors soit en milieu organique, éventuellement en présence d'un accepteur d'acide tel qu'une base organique azotée (par exemple une trialcoylamine, une pyridine, le diaza-1,8 bicyclo [5.4.0] undecène-7 ou le diaza-1,5 bicyclo [4.3.0] nonème-5), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino terreux à une température comprise entre 0 et 40°C. Il est également possible d'opérer sans solvant

à la température de fusion du mélange réactionnel. Eventuellement le dérivé réactif de l'acide peut être préparé in situ.

Selon l'invention, les produits de formule générale (I) dans laquelle le symbole R est un radical anilino, peuvent être préparés par action d'isocyanate de phényle sur une amine de formule générale (III) dans laquelle R' représente un atome d'halogène, un radical phényle, bromo-3 phényle ou anilino.

On opère généralement dans un solvant organique tel qu'un éther (tétrahydrofuranne, dioxanne par exemple), un solvant chloré (chlorure de méthylène, dichloréthane, chloroforme par exemple) ou un amide (diméthylformamide par exemple), à une température comprise entre 0 et 80°C.

Les acides de formule générale (II) peuvent être préparés par application des méthodes ci-dessous ou par analogie avec ses méthodes:

- lorsque le symbole R représente un radical alcoyloxyphényle selon la méthode décrite par S. M. Mac Elvain et T. P. Carney, J. Amer. Chem. Soc., 68, 2592 (1946).
- lorsque le symbole R représente un radical alcoyloxycarbonylphényle selon les méthodes décrites par K. G. Rutherford et coll., J. Org. Chem., 28, 582 (1963), par B. H. Chase J. Chem. Soc. 1334 (1963) ou par S. Kasina, J. Pharm. Sci., 63, 1155 (1974);
- lorsque R est un radical trialcoyloxyphényle, selon la méthode décrite par E. Kasztreiner et coll., Biochem. Pharmacology, 11, 651 (1962).

Les amines de formule générale (III) dans laquelle le symbole R' représente un radical phénoxy, chloro-4 phénoxy, anilino ou pyrrolidinyle-1, peuvent être préparées à partir de l'amine correspondante de formule générale (III) dans laquelle R' est un atome d'halogène (de préférence un atome de chlore), par action respectivement du phénol, du chloro-4 phénol, de l'aniline ou de la pyrrolidine.

Lorsque l'on veut obtenir une amine de formule générale (III) dans laquelle R' est un radical phénoxy ou chloro-4 phénoxy, on opère en milieu basique ou en présence de l'alcoolate correspondant.

La réaction s'effectue généralement en présence d'une base forte (soude, potasse, hydroxyde d'ammonium quaternaire, éthylate de sodium par exemple), à une température comprise entre 50 et 150°C, ou bien en présence de l'alcoolate correspondant (par exemple l'alcoolate de sodium), dans un solvant tel qu'un amide (diméthylformamide par exemple) ou un éther (tétrahydrofuranne, diméthoxyéthane par exemple), ou sans solvant, en présence d'un excès de dérivé hydroxylé à une témperature comprise entre 70°C et la température de reflux du mélange réactionnel.

Lorsque l'on met en oeuvre l'alcolate, celui-ci est obtenu préalablement par action du sodium sur l'alcool à une température comprise entre 20 et 80°C, ou par action de l'hydrure de sodium à une température comprise entre 0 et 20°C dans un solvant tel que le diméthylformamide, le diméthoxyéthane, ou le tétrahydrofuranne. Il n'est pas nécessaire d'isoler l'alcoolate obtenu pour le mettre en œuvre dans la réaction suivante.

Lorsque l'on veut obtenir une amine de formule générale (III) dans laquelle R' est un radical anilio ou pyrrolidinyle-1, la réaction s'effectue avec ou sans solvant à une température comprise entre 50 et 150°C. A titre de solvant on utilise avantageusement le diméthylfomamide, le dichlorométhane, ou le tétrahydrofuranne.

Les amines de formule générale (III) peuvent également être préparés par application des méthodes décrites ci-après dans les exemples ou par application ou par analogie avec les méthodes décrites par:

- S. Carboni, Gazz. Chim. Italiana, 96, 1456 (1966);
- J. F. Harper et D. G. Wibberley, J. Chem. Soc., (C) 2985 (1971).

Les amines de formule générale (III) dans laquelle R' est un radical phényle substitué peuvent être obtenues par action d'un formiate sur le dérivé correspondant de l'acétophénone, en présence de sodium, suivi par la réaction avec la diamino-2, 6 pyridine en milieu acide.

On opère généralement dans un solvant organique anhydre tel qu'un hydrocarbure aromatique (toluène par exemple) à une température comprise entre 10 et 40°C, puis à une température comprise entre 70 et 100°C.

Lorsque l'on veut obtenir une amine de formule générale (III) dans laquelle R' est un radical phényle substitué par 2 atomes de fluor, le dérivé d'acétophénone utilisé comme produit de départ peut être préparé selon les méthodes décrites par:

- J. T. Minore et coll., J. Org. Chem., 17, 1425 (1952),
- M. G. Belsham et coll., J. C. S. Perkins trans. II, 119 (1974) ou
- S. G. Malkevich et coll., Plasliches Kie Massy, 4, 1 (1960) [Chem. Abst. 55 421i].

Lorsque l'on veut obtenir une amine de formule générale (III) dans laquelle R' est un radical phényl substitué par 2 atomes de brome, le dérivé d'acétophénone utilisé comme produit de départ peut être préparé selon les méthodes décrites par:

- D. W. Mathieson et coll., J. Chem. Soc., 1133 (1949) ou
- D. E. Pearson et coll., J. Org. Chem., 23, 1412 (1958).

Les nouveaux amides selon la présente invention peuvent être purifiés le cas échéant, par des méthodes physiques telles que la cristillisation ou la chromatograhphie.

Les produits de formule générale (I) présentent des propriétés immunostimulantes particulièrement intéressantes.

L'activité immunostimulante a été mise en évidence in vitro à des concentrations molaires comprises entre $10^{-5}$ et $10^{-6}$ dans la stimulation de l'activité cytostatique des macrophages péritonéaux de la souris vis-à-vis des cellules tumorales $P_{815}$ selon une technique inspirée de M. I. C. Gyongyossi et coll., Cell. Immunol., 45, 1 (1979), et in vivo chez la souris, on observe une stimulation des réactions de défense contre l'infection a Klebsiella pneumoniae à des doses comprises entre 0,2 et 20 mg/kg i.p. selon une technique inspirée de M.A. Parant et coll., Infect. Immun., 27, 826 (1980).

Un avantage de l'activité immunostimulante des produits est leur utilisation comme agent anti-infectieux ayant notamment la possibilité de lutter contre des germes devenus résistants aux antibiotiques traditionnels.

Par ailleurs, les produits selon l'invention présentent une faible toxicité: leur $DL_{50}$ par voie orale chez la souris est soit comprise entre 300 et 900 mg/kg, soit supérieure à 900 mg/kg.

D'un intérêt particulier sont les produits de formule générale (I) dans laquelle

1) soit le symbole R représente un radical quinolyle-2, dialcoylamino-4 phényle, dichlorophényle, trialcoyloxyphényle, benzyle, styryle ou benzoyle et

le symbole R' représente un atome de chlore ou un radical phényle, ou bien

le symbole R' représente un radical phénoxy lorsque R est un radical bromophényle, ou bien

le symbole R' représente un atome de chlore lorsque R est un radical bromophényle, trifluorométhylphényle ou anilino, ou bien

le symbole R' représente un radical bromo-3 phényle lorsque R est un radical anilino,

2) soit le symbole R représente un radical phényle ou alcoyloxycarbonyle et le symbole R' représente un radical phényle, chloro-4 phénoxy, anilino ou pyrrolidinyle-1, ou bien

le symbole R' représente un radical chloro-2 (ou -4) phényle, bromo-3 (ou -4) phényle, ou alcoyloxy-4 phényle lorsque R est un radical phényle,

3) soit le symbole R représente un radical phényle substitué en position -3 par un atome d'halogène et le symbole R' représente un radical fluoro-3 (ou -4) phényle, brome-3 (ou -4) phényle, chlore-4 phényle, alcoyloxy-2 (ou -3) phényle, alcoyl-3 phényle ou difluoro (ou dichloro ou dibromo) -3, 4 phényle, les radicaux cités ci-dessus étant droits ou ramifiés et contenant 1 à 4 atomes de carbone.

Et parmi ces produits, plus spécialement actifs sont les:

- N-(phényl-7 naphtyridine-1,8 yl-2) bromo-3 benzamide
- N-(phényl-7 naphtyridine-1,8 yl-2) méthoxy-4 benzamide
- N-[(pyrrolidinyl-1)-7 naphtyridine-1,8 yl-2) benzamide
- N-[(chloro-4 phényl)-7 naphtyridine-1,8 yl-2) bromo-3 benzamide
- N-[(fluoro-4 phényl)-7 naphtyridine-1,8 yl-2) bromo-3 benzamide.

Les exemples suivants donnés à titre non limitatif, illustrent la présente invention.

*Exemple 1*

A une solution de 3 g d'acide bromo-3 benzoïque dans 50 $cm^3$ de tétrahydrofuranne anhydre, on ajoute 2,4 de N,N'carbonyldiimidazole. On observe un dégagement gazeux immédiat. Le mélange est agité pendand 2 heures à une température voisine de 20°C, jusqu'à fin du dégagement gazeux. On ajoute ensuite 2,4 g d'amino-2 phényl-7 naphtyridine-1,8 et on chauffe à reflux pendant 20 heures. Le mélange est versé dans 500 $cm^3$ d'eau distillé. Le précipité formé est séparé par filtration, lavé à l'eau et séché à l'air.

Le produit obtenu (3,8 g - F=170-175°C) est dissous dans 400 $cm^3$ d'acétonitrile bouillant. Après 4 heures de refroidissement à 4°C, le solide cristallisé est séparé par filtration, lavé par 3 fois 10 $cm^3$ d'acétonitrile et séché à 40°C sous pression réduite (0,07 kPa). On obtient 3,2 g de N-(phényl-7 naphtyridine-1,8 yl-2) bromo-3 benzamide fondant à 175°C.

L'amino-2 phényl-7 naphtyridine-1,8 peut être préparée par application de la méthode décrite par J. F. Harper, D. G. Wibberley, J. Chem. Soc. (C) 2985(1971).

*Exemple 2*

On opère d'une manière analogue à celle décrite à l'exemple 1, mais à partir de 10,4 g d'acide quinoléine carboxylique-2, de 13 g de N,N'-carbonyldiimidazole et de 7,2 g d'amino-2 chloro-7 naphtyridine-1,8. Après refroidissement à 4°C, le solide cristallisé est séparé par filtration, lavé par 5 fois 50 $cm^3$ d'eau distillé et séché à l'air. Le produit obtenu (7,4 g - F=260°C) est dissous dans un mélange de 200 $cm^3$ de diméthylformamide et de 200 $cm^3$ de méthanol. Après 3 heures de refroidissement à 4°C, le solide cristallisé est séparé par filtration, lavé par 3 fois 15 $cm^3$ de méthanol et séché à 35°C sous pression réduite (0,07 kPa). On obtient 3,6 g de N-(chloro-7 naphtyridine-1,8 yl-2) quinoléine carboxamide-2 fondant à 275°C.

*Exemple 3*

On opère d'une manière analogue à celle décrite à l'exemple 1, mais à partir de 6 g d'acide bromo-3 benzoïque de 4,9 g de N,N'-carbonyldiimidazole et de 3,6 g d'amino-2 chloro-7 naphtyridine-1,8. Le mélange réactionnel est versé dans 100 $cm^3$ d'eau distillé et extrait par 200 $cm^3$ de chlorure de méthylène. Après décantation et séchage sur sulfate de magnésium, la solution chlorométhylénique est concentrée à sec sous pression réduite (4 kPa) pour obtenir 10,2 g d'un solide qui est purifié par chromatographie sur une colonne de 3 cm de diamètre contenant 200 g de silice (0,063-0,2 mm) en éluant par un mélange (98-2 en volumes) de chlorure de méthylène-méthanol. On recueille des fractions de 40 $cm^3$, les fractions 11 à 16 sont réunies et concentrées à sec sous pression réduite (4 kPa) pour obtenir 7,1 g d'un solide fondant à 222°C.

Ce produit est dissous dans 300 $cm^3$ d'éthanol bouillant. Après 2 heures de refroidissement à 4°C, le solide cristallisé est séparé par filtration, lavé par 3 fois 20 $cm^3$ d'éthanol, 2 fois 20 $cm^3$ d'oxyde de diéthyle et séché à 40°C sous pression réduite (4 kPa). On obtient 8,7 g de N-(chloro-7 naphtyridine-1,8 yl-2) bromo-3 benzamide fondant à 224°C.

*Exemple 4*

On opère d'une manière analogue à celle décrite à l'exemple 1, mais à partir de 6 g d'acide bromo-3 benzoïque de 4,9 g de N,N'-carbonyldiimidazole et de 5,9 g d'amino-2 phénoxy-7 naphtyridine-1,8. Le produit obtenu par précipitation dans l'eau (9,8 g - F=188-192°C) est dissous dans 300 $cm^3$ d'acétonitrile. Après 1 heure de refroidissement à 4°C, le solide cristallisé est séparé par filtration, lavé par 3 fois 10 $cm^3$ d'acétonitrile et séché à 40°C sous pression réduite (0,07 kPa). On obtient 5,4 g de N-(phénoxy-7 naphtyridine-1,8 yl-2) bromo-3 benzamide fondant à 194°C.

L'amino-2 phénoxy-7 naphtyridine-1,8 peut être préparée de la façon suivante:

On chauffe pendant 20 heures à 120°C un mélange composé de 27 g d'amino-2 chloro-7 naphtyridine-1,8 de 141 g de phénol et de 19,8 g de potasse en pastilles à 85%. Le mélange obtenu est repris par 300 $cm^3$ d'une solution aqueuse de soude 4N et extrait par 250 $cm^3$ de chlorure de méthylène.

La phase aqueuse est extraite à nouveau par 2 fois 200 $cm^3$ de chlorure de méthylène. Les extraits organiques sont lavés par 2 fois 150 $cm^3$ de soude 4N puis 250 $cm^3$ d'eau distillée, séchés sur sulfate de magnésium et concentrés à sec à 40 °C sous pression réduite (4 kPa).

Le produit obtenu (30,5 g; F=190-194°C) est dissous dans 400 $cm^3$ d'acétonitrile bouillant. Après 2 heures de refroidissement à 4°C, le solide cristallisé est séparé par filtration, lavé par 20 $cm^3$ d'acétonitrile et séché à 45°C, sous pression réduite (0,07 kPa). On obtient 23,4 g d'amino-2 phénoxy-7 naphtyridine-1,8 fondant à 196°C.

*Exemple 5*

On opère d'une manière analogue à celle décrite à l'exemple 1, mais à partir de 1,6 g d'acide chloro-3 benzoïque de 1,6 g de N,N'-carbonyldiimidazole et de 1,6 g d'amino-2 phényl-7 naphtyridine-1,8. Le produit obtenu par précipitation dans l'eau (2,2 g; F environ 160°C) est dissous dans 200 $cm^3$ de d'acétonitrile bouillant. Après 4 heures de refroidissement à 4°C, le solide cristallisé est séparé par filtration, lavé par 3 fois 10 $cm^3$ d'acétonitrile et séché à 40°C sous pression réduite (0,07 kPa). On obtient 1,5 g de N-(phényl-7 naphtyridine-1,8 yl-2) chloro-3 benzamide fondant à 175°C.

L'amino-2 phényl-7 naphtyridine-1,8 peut être préparée par application de la méthode décrite par J. F. Harper, D. G. Wibberley, J. Chem. Soc. (C) 2985(1971).

*Exemple 6*

On opère d'une manière analogue à celle décrite à l'exemple 1, mais à partir de 10 g d'acide trifluorméthyl-3 benzoïque, de 8,4 g de N,N'-carbonyldiimidazole et de 5,9 g d'amino-2 chloro-7 naphtyridine-1,8. Le produit obtenu par précipitation dans l'eau (11,5 g; F=235°C) est dissous dans 250 $cm^3$ d'acétonitrile bouillant. Après 4 heures de refroidissement à 4°C, le solide cristallisé est séparé par filtration, lavé par 2 fois 20 $cm^3$ d'acétonitrile et séché à 45°C sous pression réduite (0,07 kPa). On obtient 9 g de N-(chloro-7 naphtyridine-1,8 yl-2) trifluorméthyl-4 benzamide fondant à 236°C.

*Exemple 7*

On opère d'une manière analogue à celle décrite à l'exemple 11, mais à partir de 2,7 g d'acide trifluorméthyl-4 benzoïque de 2,3 g de N,N'-carbonyldiimidazole et de 2,4 g d'amino-2 (fluoro-4 phényl) naphtyridine-1,8. Le produit obtenu par précipitation dans l'eau (2,1 g) est dissous dans 50 $cm^3$ de méthanol bouillant. Après 2 heures de refroidissment à 4°C le solide cristallisé est séparé par filtration, lavé par 3 fois 5 $cm^3$ de méthanol et séché à 40°C sous pression réduite (0,07 kPa). On obtient 1,3 g de N-[(fluoro-4 phényl)-7 naphtyridine-1,8 yl-2] trifluorméthyl-4 benzamide fondant à 198°C.

L'amino-2 (fluoro-4 phényl)-7 naphtyridine-1,8 peut être préparée de la façon suivante:

On opère d'une manière analogue à celle décrite à l'exemple 11, mais à partir de 2,3 g de sodium, de 13,8 g de fluoro-4 acétophénone et de 11 g de formiate d'éthyle. Les 17,8 g de solide obtenus sont alors ajoutés à 11 g de diamino-2,6 pyridine. Le produit obtenu après hydrolyse et neutralisation à la soude est séparé par filtration, lavé par 6 fois 100 $cm^3$ d'eau distillé et séché à l'air. On obtient anisi 17,8 g d'amino-2 (fluoro-4 phényl)-7 naphtyridine fondant à 175°C.

*Exemple 8*

On opère d'une manière analogue à celle décrite à l'exemple 11, mais à partir de 2,7 g d'acide trifluorméthyl-4 benzoïque de 2,3 g de N,N'-carbonyldiimidazole et de 2,5 g d'amino-2 (méthoxy-4 phényl) naphtyridine-1,8. Le produit obtenu par précipitation dans l'eau (3,3 g; F=144-146°C) est dissous dans 100 $cm^3$ d'acétonitrile bouillant. Après 3 heures de refroidissement à 4°C, le solide cristallisé est séparé par filtration, lavé par 2 fois 10 $cm^3$ d'acétonitrile et séché à 40°C sous pression réduite (0,07 kPa). On obtient 2,4 g de N-[(méthoxy-4 phényl)-7 naphtyridine-1,8 yl-2] trifluorméthyl-4 benzamide fondant à 148°C.

*Exemple 9*

A une suspension de 7,2 g d'amino-2 chloro-7 naphtyridine-1,8 et de 4,4 g de téréphtalate de méthyle et de potassium dans 80 $cm^3$ de toluène, on ajoute en 10 minutes 1,55 g de chlorure de phosphoryle. Le mélange réactionnel est ensuite chauffé pendant 5 heures à reflux. La suspension obtenue est filtrée, lavée par 3 fois 20 $cm^3$ de toluène, 3 fois 20 $cm^3$ d'eau distillée, 3 fois 20 $cm^3$ d'éthanol et séché à 50°C sous pression réduite (0,07 kPa). Le solide obtenu (9 g; fondant au-dessus de 260°C) est purifié par chromatographie sur une colonne de 4 cm de diamètre contenant 150 g de silice (0,040-0,063 mm) en éluant par un mélange de chlorure de méthylène et de méthanol (98-2 en volumes). On recueille des fractions de 50 $cm^3$, les fractions 21 à 72 sont concentrées à sec sous pression réduite (4 kPa) pour obtenir 5,1 g d'un solide qui est dissous dans 1000 $cm^3$ de propanol-1 bouillant. Après 3 heures de refroidissement à 4°C, le solide cristallisé est séparé par filtration, lavé par 3 fois 20 $cm^3$ de propanol-1, 3 fois 20 $cm^3$ d'oxyde de diéthyle puis séché à 50°C sous pression réduite (0,07 kPa). On obtient 4,3 g de N-(chloro-7 naphtyridine-1,8 yl-2) méthoxycarbonyl-4 benzamide fondant à 275°C.

Le téréphtalate de méthyle et de potassium peut être préparé par la méthode décrite par B. W. Hotten Ind. Eng. Chem., Int. Ed., 49, 1961 (1957).

*Exemple 10*

On opère d'une manière analogue à celle décrite à l'exemple 1, mais à partir de 14,9 g d'acide diméthylamino-4 benzoïque de 14,6 g de N,N'-carbonyldiimidazole et de 10,8 g d'amino-2 chloro-7 naphtyridine-1,8. Le produit brut obtenu par filtrationdu mélange réactionnel (6,4 g; F=248°C) est dissous dans 95 $cm^3$ de diméthylformamide bouillant. Après 2 heures de refroidissement à 20°C, on ajoute 95 $cm^3$ d'eau. Le solide cristallisé est séparé par filtration, lavé par 2 fois 10 $cm^3$ d'un mélange diméthylformamide-eau (50-50 en volumes), et séché à 45°C sous pression réduite (0,07 kPa). On obtient 6,2 g de N-(chloro-7 naphtyridine-1,8 yl-2) diméthylamino-4 benzamide fondant à 248°C.

*Exemple 11*

A une solution de 3,3 g d'acide diméthylamino-4 ben-

zoïque dans 60 $cm^3$ de tétrahydrofuranne anhydre, on ajoute 3,2 g de N,N'-carbonyldiimidazole. On observe un dégagement gazeux immédiat. Le mélange est agité pendant 2 heures à 20°C, jusqu'à fin du dégagement gazeux. On ajoute ensuite 3,8 g d'amino-2 (méthoxy-4 phényl)-7 naphtyridine-1,8 et on chauffe à reflux pendant 20 heures. Le mélange est versé dans 500 $cm^3$ d'eau distillée. Le précipité formé est séparé par filtration, lavé à l'eau et séché à l'air.

Le produit obtenu (3 g; F environ 270°C) est dissous dans un mélange de 150 $cm^3$ de diméthylfomamide et 100 $cm^3$ d'acétonitrile. Après 4 heures de refroidissement à 4°C, le solide cristallisé est séparé par filtration, lavé par 3 fois 10 $cm^3$ d'acétonitrile, et séché à 40°C sous pression réduite (0,07 kPa). On obtient 1,2 g de N-[(méthoxy-4 phényl)-7 naphtyridine-1,8 yl-2] diméthylamino-4 benzamide fondant à 275°C.

L'amino-2 (méthoxy-4 phényl)-7 naphtyridine-1,8 peut être préparée de la façon suivante:

Dans une suspension de 2,3 g de sodium en petits morceaux dans 600 $cm^3$ de toluène anhydre, on verse un mélange de 15 g de méthoxy-4 acétophénone et de 11 g de formiate d'éthyle. Le mélange réactionnel est maintenu à une température voisine de 25°C, et agité pendant 20 heures. On ajoute 200 $cm^3$ d'éther isopropylique à la suspension obtenue.

Le solide (17,5) obtenu par filtration et séchage à l'air, est ajouté en 30 minutes à une solution de 10,4 g de diamino-2,6 pyridine dans 180 $cm^3$ d'acide phosphorique (d=1,70). Le mélange réactionnel est chauffé à 85°C pendant 2 heures, agité 20 heures à une température voisine de 25°C puis versé dans un mélange de 1000 $cm^3$ d'eau distillée et 500 g de glace. Le précipité formé après neutralisation à la soude 10N est séparé par filtration, lavé par 5 fois 150 $cm^3$ d'eau distillée et séché à l'air.

Le produit obtenu (13 g, F environ 190°C) est dissous dans un mélange de 100 $cm^3$ de diméthylformamide et 100 $cm^3$ d'acétonitrile. Après 3 heures de refroidissement à 4°C, le solide cristallisé est séparé par filtration, lavé par 3 fois 15 $cm^3$ d'acétonitrile et séché à l'air. On obtient 5,4 g d'amino-2 (méthoxy-4 phényl)-7 naphtyridine-1,8 fondant à 220°C.

*Exemple 12*

On opère d'une manière analogue à celle décrite à l'exemple 1, mais à partir de 15,3 g d'acide dichloro-3,4 benzoïque, de 12,9 g de N,N'-carbonyldiimidazole et de 8,9 g d'amino-2 chloro-7 naphtyridine-1,8. Le produit obtenu par précipitation dans l'eau (17,6 g; F= 228°C) est dissous dans 900 $cm^3$ d'éthanol bouillant. Après 2 heures de refroidissement à 4°C, le solide cristallisé est séparé par filtration, lavé par 2 fois 30 $cm^3$ d'oxyde d'isopropyle et séché à 40°C sous pression réduite (0,07 kPa). On obtient 9,2 g de N-(chloro-7 naphtyridine-1,8 yl-2) dichloro-3,4 benzamide fondant à 230°C.

*Exemple 13*

On opère d'une manière analogue à celle décrite à l'exemple 1, mais à partir de 17 g d'acide triméthoxy-3,4,5 benzoïque, de 13 g de N,N'-carbonyldiimidazole et de 8,9 g d'amino-2 chloro-7 naphtyridine-1,8. Le produit obtenu par précipitation dans l'eau (10,3 g; F=90°C) est dissous dans 500 $cm^3$ d'éthanol bouillant. Après 4 heures de refroidissement à 4°C, le solide cristallisé est séparé par filtration, lavé par 20 $cm^3$ d'éthanol et séché à 40°C sous pression réduite (0,07 kPa). On obtient 8,2 g de N-(chloro-7 naphtyridine-1,8 yl-2) triméthoxy-3,4,5 benzamide fondant à 210°C.

*Exemple 14*

On opère d'une manière analogue à celle décrite à l'exemple 1, mais à partir de 10,9 g d'acide phénylacétique, de 13 g de N,N'-carbonyldiimidazole et de 10,8 g d'amino-2 chloro-7 naphtyridine-1,8. Le produit obtenu par précipitation dans l'eau (13,5 g; F=184°C) est dissous dans 210 $cm^3$ d'acétonitrile bouillant. Après 1 heure de refroidissement à 4°C, le solide cristallisé est séparé par filtration, lavé par 10 $cm^3$ d'acétonitrile et séché à 45°C sous pression réduite (0,07 kPa). On obtient 11,3 g de N-(chloro-7 naphtyridine-1,8 yl-2) phénylacétamide fondant à 187°C.

*Exemple 15*

A une suspension de 7,2 g d'amino-2 chloro-7 naphtyridine-1,8, dans 80 $cm^3$ de pyridine, on ajoute 7,3 g de chlorure de cinnamoyle. Après 2 heures d'agitation à 20°C, la suspension obtenue est versée dans 500 $cm^3$ d'eau distillée. La suspension est filtrée, lavée par 3 fois 50 $cm^3$ d'eau et séchée à 50°C sous pression réduite (0,07 kPa). Le solide obtenu (10,6 g; F=263°C) est dissous dans 1200 $cm^3$ de propanol-1 bouillant. Après 4 heures de refroidissement à 4°C, le solide cristallisé est séparé par filtration, lavé par 3 fois 50 $cm^3$ de propanol-1, 2 fois 50 $cm^3$ d'oxyde de diéthyle et séché à 50°C sous pression réduite (0,07 kPa). On obtient 11,4 g de N-(chloro-7 naphtyridine-1,8 yl-2) phényl-3 propénamide fondant à 263°C.

*Exemple 16*

On opère d'une manière analogue à celle décrite à l'exemple 1, mais à partir de 22,5 g d'acide phénylglyoxylique, de 32,4 g de N,N'-carbonyldiimidazole et de 18 g d'amino-2 chloro-7 naphtyridine-1,8. Après refroidissement du mélange réactionnel, le solide cristallisé est séparé par filtration, lavé par 5 fois 100 $cm^3$ d'eau distillée puis 2 fois 50 $cm^3$ d'éther éthylique, et séché à l'air. Le produit obtenu (14,4 g; F sup. à 250°C) est dissous dans un mélange de 100 $cm^3$ de diméthylformamide et 300 $cm^3$ de méthanol. Après 1 heures de refroidissement à 4°C, le solide cristallisé est séparé par filtration, lavé par 3 fois 50 $cm^3$ de méthanol et séché à 40°C sous pression réduite (0,07 kPa). On obtient 10,7 g de chloro-2 phénylglyoxyloylamino-7 naphtyridine-1,8 fondant à 273-275°C.

*Exemple 17*

A une solution de 17,9 g d'amino-2 chloro-7 naphtyridine-1,8 dans 600 $cm^3$ de tétrahydrofuranne anhydre, on ajoute 15,5 g d'isocyanate de phényle et chauffe à environ 60°C pendant 15 heures. Après refroidissement du mélange, le solide cristallisé est séparé par filtration, lavé par 3 fois 50 $cm^3$ d'éther éthylique et séché à l'air. On obtient 25,2 g de produit brut fondant à 270°C. 15 g de ce produit sont purifiés dans les conditions suivantes: le produit

est dissous dans un mélange de 200 $cm^3$ de diméthylformamide et 125 $cm^3$ d'acétonitrile. Après 5 heures de refroidissement à 20°C, le solide cristallisé est séparé par filtration, lavé par 3 fois 30 $cm^3$ d'acétonitrile et séché à 40°C sous pression réduite (0,07 kPa). On obtient 10,1 g de N-(chloro-7 naphtyridine-1,8 yl-2)-N'-phénylurée fondant à 276°C.

*Exemple 18*

A une solution de 1,5 g d'amino-2 (bromo-3 phényl)-7 naphtyridine-1,8 dans 45 $cm^3$ de tétrahydrofuranne anhydre, on ajoute 0,7 g d'isocyanate de phényle et on chauffe à environ 60°C pendant 15 heures. Après refroidissement du mélange, le solide cristallisé est séparé par filtration, lavé par 3 fois 10 $cm^3$ d'éther diéthylique et séché à l'air. On obtient 1,1 g de produit brut fondant à environ 260°C.

Le produit est dissous dans un mélange bouillant de 100 $cm^3$ de diméthylformamide et de 100 $cm^3$ d'acétonitrile. Après 3 heures de refroidissement à 20°C, le solide cristallisé est séparé par filtration, lavé par 3 fois 10 $cm^3$ d'acétonitrile et séché à 40°C sous pression réduite (0,07 kPa). On obtient 0,8 g de N-[(bromo-3 phényl)-7 naphtyridine-1,8 yl-2] N'-phénylurée fondant à 285°C.

*Exemple 19*

On opère d'une manière analogue à celle décrite à l'exemple 1, mais à partir de 12,6 g d'acide benzoïque, de 7,45 g de N,N'-carbonyldiimidazole et de 12,6 g d'amino-2 (chloro-4 phénoxy)-7 naphtyridine-1,8. Le produit obtenu par précipitation dans l'eau (13 g; F = 100°C) est purifié par chromatographie sur une colonne de 4 cm de diamètre contenant 200 g de silice (0,040-0,063 mm) en éluant par du chlorure de méthylène et en recueillant des fractions de 100 $cm^3$. Les fractions 10 à 25 sont concentrées au 9/10 du volume initial. Après 2 heures de refroidissement à 0°C, le solide cristallisé est séparé par filtration, lavé par 2 fois 5 $cm^3$ d'oxyde de diéthyle et séché à 40°C sous pression réduite (0,07 kPa). On obtient 4 g de N-[(chloro-4 phénoxy)-7 naphtyridine-1,8 yl-2] benzamide fondant à 148°C.

L'amino-2 (chloro-4 phénoxy)-7 naphtyridine-1,8 peut être préparée de la façon suivante:

On chauffe pendant 8 heures à 140°C un mélange composé de 1,8 g d'amino-2 chloro-7 naphtyridine-1,8, de 12,85 g de chloro-4 phénol et de 1,1 g de potasse en pastilles à 85%. Le mélange réactionnel est versé dans 200 $cm^3$ d'eau distillée et alcalinisé jusqu'à pH = 10 par de la soude 4N. La suspension obtenu est filtrée et lavée à l'eau distillée jusqu'à pH environ 7. Le solide obtenu est séché à l'air pour donner 2 g d'amino-2 (chloro-4 phénoxy)-7 naphtyridine-1,8 fondant à 177°C.

*Exemple 20*

On opère d'une manière analogue à celle décrite à l'exemple 1, mais à partir de 6,5 g d'acide benzoïque de 8,6 g de N,N'-carbonyldiimidazole et de 8,6 g d'amino-2 (pyrrolidinyl-1)-7 naphtyridine-1,8. Le produit obtenu par précipitation dans l'eau (13 g; F = 110°C) est dissous dans 85 $cm^3$ d'acétonitrile bouillant. Après 2 heures de refroidissement à 4°C, le solide cristallisé est séparé par filtration, lavé par 15 $cm^3$ d'acétonitrile et séché à 40°C sous pression réduite (0,07 kPa). On obtient 11,5 g de N-[(pyrrolidinyl-1)-7 naphtyridine-1,8 yl-2] benzamide fondant à 130°C.

L'amino-2 (pyrrolidinyl-1)-7 naphtyridine-1,8 peut être préparée de la façon suivante:

On chauffe pendant 2 heures à 90°C un mélange composé de 36 g d'amino-2 chloro-7 naphtyridine et de 250 $cm^3$ de pyrrolidine. La suspension obtenue est filtrée, lavée par 150 $cm^3$ d'oxyde de diéthyle puis 4 fois 100 $cm^3$ d'eau. 11 g du solide obtenu après séchage à l'air (37 g; F = 249°C) sont dissous dans 550 $cm^3$ d'acétonitrile bouillant. Après refroidissement à 4°C pendant 1 heure, le solide cristallisé est séparé par filtration, lavé par 20 $cm^3$ d'acétonitrile et séché à 40°C sous pression réduite (0,07 kPa). On obtient 8,3 g d'amino-2 (pyrrolidinyl-1)-7 naphtyridine-1,8 fondant à 251°C.

*Exemple 21*

On opère d'une manière analogue à celle décrite à l'exemple 24, mais à partir de 2 g d'amino-2 (chloro -2 phényl)-7 naphtyridine-1,8 et de 4 g d'anhydride benzoïque. Le produit obtenu après précipitation par l'éther éthylique (1,2 g; F=153-155°C) est dissous dans 30 $cm^3$ d'acétonitrile bouillant. Après 2 heures de refroidissement à 4°C, le solide cristallisé est séparé par filtration, lavé par 2 fois 2 $cm^3$ d'acétonitrile et séché à 40°C sous pression réduite (0,07 kPa). On obtient 0,8 g de N-[(chloro-2 phényl)-7 naphtyridine-1,8 yl-2] benzamide fondant à 156°C.

L'amino-2 (chloro-2 phényl)-7 naphtyridine-1,8 peut être préparée de la façon suivante:

On opère d'une manière analogue à celle décrite à l'exemple 11, mais à partir de 2,3 g de sodium, de 15 g de chloro-2 acétophénone et de 11 g de formiate d'éthyle. Les 20 g de solide obtenus sont alors ajoutés à 11 g de diamino-2,6 pyridine. Le produit obtenu après hydrolyse et neutralisation par la soude (21,5 g; F environ 165°C) est dissous dans 100 $cm^3$ d'acide chlorhydrique concentré (d = 1,19). Le précipité obtenu après hydrolyse avec 500 $cm^3$ d'eau distillée, est séparé par filtration, lavé par 3 fois 100 $cm^3$ de soude 0,5M puis 5 fois 50 $cm^3$ d'eau distillé et séché à l'air. On obtient 11 g d'amino-2 (chloro-2 phényl)-7 naphtyridine fondant à 193°C.

*Exemple 22*

On opère d'une manière analogue à celle décrite à l'exemple 24, mais à partir de 1 g d'amino-2 (chloro-4 phényl)-7 naphtyridine-1,8 et de 2,2 g d'anhydride benzoïque. Le produit obtenu après précipitation par l'éther éthylique (1,1 g; F environ 100°C) est purifié par chromatographie sur une colonne de 30 mm de diamètre contenant 50 g de silice (0,04-0,06 mm) en éluant par du chlorure de méthylène en recueillant des fractions de 15 $cm^3$. Après concentration à sec des fractions 12 à 28 à 40°C sous pression réduite (4 kPa), on obtient 0,6 g d'un solide fondant à 155°C. Ce produit est dissous dans 3 $cm^3$ d'isopropanol bouillant. Après 2 heures de refroidissement à 4°C, le solide cristallisé est séparé par filtration, lavé par 0,5 $cm^3$ d'isopropanol et séché à 40°C sous pression réduite (0,07 kPa). On

obtient 0,5 g de N-[(chloro-4 phényl)-7 naphtyridine-1,8 yl-2] benzamide fondant à 156-158°C.

L'amino-2 (chloro-4 phényl)-7 naphtyridine-1,8 peut être préparée de la façon suivante:

On opère d'une manière analogue à celle décrite à l'exemple 11, mais à partir de 2,3 g de sodium, de 15,5 g de chloro-4 acétophénone et de 11 g de formiate d'éthyle. Les 20,4 g de solide obtenus sont alors ajoutés à 11,6 g de diamino-2,6 pyridine. Le produit obtenu après hydrolyse et neutralisation par la soude est séparé par filtration, lavé par 6 fois 100 $cm^3$ d'eau distillée à une température voisine de 50°C puis séché à l'air. On obtient ansi 22,6 g d'amino-2 (chloro-4 phényl)-7 naphtyridine-1,8 fondant à environ 130°C.

*Exemple 23*

On opère d'une manière analogue à celle décrite à l'exemple 24, mais à partir de 1 g d'amino-2 (bromo-3 phényl)-7 naphtyridine-1,8 et de 2,2 g d'anhydride benzoïque. Le produit obtenu par précipitation à l'éther éthylique (1,2 g; F environ 110°C) est purifié par chromatographie sur une colonne de 30 mm de diamètre contenant 50 g de silice (0,04-0,06 mm) en éluant par du chlorure de méthylène en recueillant des fractions de 20 $cm^3$. Après concentration à sec des fractions 15 à 32 à 40°C sous pression réduite (4 kPa), on obtient 0,7 g d'un solide fondant à 202-204°C. Ce produit est dissous dans 3 $cm^3$ de chlorure de méthylène bouillant. Après 2 heures de refroidissement à 4°C, le solide cristallisé est séparé par filtration, lavé par 0,5 $cm^3$ de chlorure de méthylène et séché à 40°C sous pression réduite (0,07 kPa). On obtient 0,5 g de N-[(bromo-3 phényl)-7 naphtyridine-1,8 yl-2] benzamide fondant à 204-206°C.

L'amino-2 (bromo-3 phényl)-7 naphtyridine peut être préparée de la façon suivante:

On opère d'une manière analogue à celle décrite à l'exemple 11, mais à partir de 2,3 g de sodium, de 20 g de bromo-3 acétophénone et de 11 g de formiate d'éthyle. Les 25 g de solide obtenu sont alors ajoutés à 11,6 g de diamino-2,6 pyridine. Le produit obtenu après hydrolyse et neutralisation à la soude (8,6; F=160°C) est dissous dans 100 $cm^3$ d'acide chlorhydrique concentré (d=1,19), précipité par 400 $cm^3$ d'eau distillée, séparé par filtration, lavé par 6 fois 40 $cm^3$ d'eau distillé et séché à l'air.

On obtient 6,2 g d'amino-2 (bromo-3 phényl)-7 naphtyridine-1,8 fondant à 165-166°C.

*Exemple 24*

0,8 g d'amino-2 (méthoxy-4 phényl)-7 naphtyridine-1,8 sont ajoutés à 2 g d'anhydride benzoïque. Le mélange réactionnel est chauffé pendant 30 minutes à 140°C. Après refroidissement à 25°C, 15 $cm^3$ d'éther éthylique sont ajoutés. Le précipité formé est séparé par filtration, lavé par 3 fois 5 $cm^3$ d'éther éthylique et séché à l'air.

Le produit obtenu (1,2 g; F environ 140°C) est purifié par chromatographie sur une colonne de 30 mm de diamètre contenant 40 g de silice (0,04-0,06 mm) en éluant par du chlorure de méthylène en recueillant des fractions de 15 $cm^3$. Après concentration à sec des fractions 10 à 25 à 40°C sous pression réduite (4 kPa), on obtient 0,6 g d'un solide fondant à 185°C. Ce produit est dissous dans 6 $cm^3$ d'acétonitrile bouillant. Après 2 heures de refroidissement à 4°C, le solide cristallisé est séparé par filtration, lavé par 2 fois 0,5 $cm^3$ d'acétonitrile et séché à 40°C sous pression réduite (0,07 kPa). On obtient 0,45 g de N-[(méthoxy-4 phényl)-7 naphtyridine-1,8 yl-2] benzamide fondant à 185-187°C.

*Exemple 25*

On opère d'une manière analogue à celle décrite à l'exemple 24, mais à partir de 2,5 g d'amino-2 (dichloro-3,4 phényl)-7 naphtyridine-1,8 et de 6 g d'anhydride benzoïque. Le produit obtenu par précipitation à l'éther éthylique (1,8 g; F environ 100°C) est dissous dans 25 $cm^3$ d'acétonitrile bouillant. Le produit cristallisé obtenu après refroidissment est séparé par filtration, dissous dans un mélange de 2 $cm^3$ d'éthanol et 5 $cm^3$ d'acétonitrile bouillant. Après 3 heures de refroidissement à 4°C, le solide cristallisé est séparé par filtration, lavé par 2 fois 1 $cm^3$ d'acétonitrile et séché à 40°C sous pression réduite (0,07 kPa). On obtient 0,55 g de N-[(dichloro-3,4 phényl)-7 naphtyridine-1,8 yl-2] benzamide fondant à environ 110°C puis 205°C.

L'amino-2 (dichloro-3,4 phényl)-7 naphtyridine-1,8 peut être préparée de la façon suivante:

On opère d'une manière analogue à celle décrite à l'exemple 11, mais à partir de 2,3 g de sodium, de 19 g de dichloro-3,4 acétophénone et de 11 g de formiate d'éthyle. Le solide alors obtenu (22,7) est ajoutés à 11,6 g de diamino-2,6 pyridine. Le produit obtenu après hydrolyse et neutralisation par la soude 10N (22,4 g; F environ 140°C) est dissous dans 200 $cm^3$ d'acide chlorhydrique concentré (d=1,19). Le précipité obtenu après hydrolyse avec 300 $cm^3$ d'eau distillée puis neutralisation à la soude 10N, est séparé par filtration, lavé par 5 fois 50 $cm^3$ d'eau distillé et séché à l'air. On obtient 14,6 g d'amino-2 (dichloro-3,4 phényl)-7 naphtyridine-1,8 fondant à 210°C.

*Exemple 26*

On opère d'une manière analogue à celle décrite à l'exemple 1, mais à partir de 7,6 g d'acide méthoxy-4 benzoïque de 9,7 g de N,N'-carbonyldiimidazole et de 13,3 g d'amino-2 phényl-7 naphtyridine-1,8. Le mélange réactionnel est versé dans 400 $cm^3$ d'eau distillé et extrait par 2 fois 200 $cm^3$ d'acétate d'éthyle. Après lavage à l'eau et séchage sur sulfate de magnésium, les extraits organiques sont concentrés à sec sous pression réduite (4 kPa) pour obtenir 14,5 g d'une huile épaisse. L'huile obtenue est purifiée par chromatographie sur une colonne de 4,2 cm de diamètre contenant 300 g de silice (0,040-0,063 mm) en éluant par du chlorure de méthylène. On recueillant des fractions de 100 $cm^3$; les fractions 12 à 25 sont concentrées à sec sous pression réduite (4 kPa) pour obtenir 13, 4 g d'un solide fondant vers 60°C qui est dissous dans 425 $cm^3$ d'acétate d'éthyle bouillant. Après 2 heures de refroidissement à 4°C, le solide obtenu est séparé par filtration, lavé par 2 fois 15 $cm^3$ d'acétate d'éthyle et séché à 40°C sous pression réduite (0,07 kPa). On obtient 7,5 g de N-(phényl-7 naphtyridine-1,8 yl-2) méthoxy-4 benzamide fondant à 187°C.

L'amino-2 phényl-7 naphtyridine-1,8 peut être préparée selon la méthode décrite par J. F. Harper, D. G. Wibberley, J. Chem. Soc. (C) 2985 (1971).

*Exemple 27*

On opère d'une manière analogue à celle décrite à l'exemple 1, mais à partir de 6,1 g d'acide méthoxy-4 benzoïque, de 6,5 g de N,N'-carbonyldiimidazole et de 7 g d'amino-2 anilino-7 naphtyridine-1,8. Le produit obtenu par précipatation dans l'eau (12 g) est dissous dans 140 cm³ d'éthanol bouillant. Après 2 heures de refroidissement à 4°C le solide cristallisé est séparé par filtration, lavé par 10 cm³ d'éthanol et séché à 45°C sous pression réduite (0,07 kPa). On obtient 4,5 g de N-(anilino-7 naphtyridine-1,8 yl-2) méthoxy-4 benzamide fondant à 145°C.

L'amino-2 anilino-7 naphtyridine-1,8 peut être obtenue de la façon suivante:

Une suspension de 9 g d'amino-2 chloro-7 naphtyridine-1,8 dans 19,6 g d'aniline est chauffé pendant 19 heures à 120 °C. La suspension obtenue est filtrée, lavée avec 3 fois 15 cm³ d'oxyde de diéthyle et séchée à l'air. Le solide brut obtenu (14 g; F=190°C) est ensuite dissous dans 700 cm³ d'éthanol bouillant. Après 2 heures de refroidissement à 4°C, le produit cristallisé est séparé par filtration, lavé par 25 cm³ d'éthanol et séché à l'air. On obtient 7,2 g d'amino-2 anilino-7 naphtyridine-1,8, fondant à environ 250°C.

*Exemple 28*

On opère d'une manière analogue à celle décrite à l'exemple 11, mais à partir de 2,8 g d'acide bromo-3 benzoïque, de 2,3 g de N,N'-carbonyldiimidazole et de 2,4 g d'amino-2 (fluoro-3 phényl)-7 naphtyridine-1,8. Le produit obtenu par précipitation dans l'eau (3,5 g; F environ 195°C) est dissous dans un mélange de diméthylformamide et 200 cm³ de 20 cm³ d'acétonitrile bouillant. Après 4 heures de refroidissement à 4°C le solide cristallisé est séparé par filtration, lavé par 3 fois 10 cm³ d'acétonitrile et séché à 40°C sous pression réduite (0,07 kPa). On obtient 2,3 g de N-[(fluoro-3 phényl)-7 naphtyridine-1,8 yl-2] bromo-3 benzamide fondant à 208°C.

L'amino-2 (fluoro-3 phényl)-7 naphtyridine-1,8 peut être préparée de la façon suivante:

On opère d'une manière analogue à celle décrite à l'exemple 11, mais à partir de 2,5 g de sodium, de 15 g de fluoro-3 acétophénone et de 12,6 g de formiate d'éthyle. Les 18,8 g de solide obtenus sont alors ajoutés à 12,1 g de diamino-2,6 pyridine. Le produit obtenu après hydrolyse et neutralisation par la soude (22 g; F environ 140°C) est dissous dans 250 cm³ de diméthylfomamide. Le produit obtenu par précipitation dans l'eau est séparé par filtration, lavé par 2 fois 100 cm³ d'eau distillé et séché à l'air.

On obtient 14,8 g d'amino-2 (fluro-3 phényl)-7 naphtyridine-1,8 fondant à 160°C.

*Exemple 29*

On opère d'une manière analogue à celle décrite à l'exemple 11, mais à partir de 2,1 g d'acide bromo-3 benzoïque de 1,6 g de N,N'-carbonyldiimidazole et de 1,7 g d'amino-2 (fluoro-4 phényl)-7 naphtyridine-1,8. Le produit obtenu par précipitation dans l'eau (2 g) est dissous dans 200 cm³ de propanol bouillant. Après 4 heures de refroidissement à 4°C le solide cristallisé est séparé par filtration, lavé par 3 fois 10 cm³ de propanol et séché à 40°C sous pression réduite (0,07 kPa). On obtient 1,4 g de N-[(fluoro-4 phényl)-7 naphtyridine-1,8 yl-2] bromo-3 benzamide fondant à 210°C.

*Exemple 30*

On opère d'une manière analogue à celle décrite à l'exemple 11, mais à partir de 1,6 g d'acide chloro-3 benzoïque, de 1,6 g de N,N'-carbonyldiimidazole et de 1,8 g d'amino-2 (chloro-4 phényl)-7 naphtyridine-1,8. Le produit obtenu par précipitation dans l'eau (2,2 g; F environ 190°C) est dissous dans 400 cm³ de propanol bouillant. Après 4 heures de refroidissement à 4°C le solide cristallisé est séparé par filtration, lavé par 3 fois 5 cm³ de propanol et séché à 40°C sous pression réduite (0,07 kPa). On obtient 0,85 g de N-[(chloro-4 phényl)-7 naphtyridine-1,8 yl-2] chloro-3 benzamide fondant à 222-224°C.

*Exemple 31*

On opère d'une manière analogue à celle décrite à l'exemple 11, mais à partir de 2,1 g d'acide bromo-3 benzoïque, de 1,6 g de N,N'-carbonyldiimidazole et de 1,8 g d'amino-2 (chloro-4 phényl)-7 naphtyridine-1,8. Le produit obtenu par précipitation dans l'eau (2,2 g; F = 210°C) est dissous dans un mélange de 20 cm³ de diméthylformamide et 100 cm³ d'acétonitrile bouillant. Après 4 heures de refroidissement à 4°C le solide cristallisé est séparé par filtration, lavé par 3 fois 10 cm³ d'acétonitrile et séché à 40°C sous pression réduite (0,07 kPa). On obtient 1,4 g de N-[(chloro-4 phényl)-7 naphtyridine-1,8 yl-2] bromo-3 benzamide fondant à 220-224°C.

*Exemple 32*

On opère d'une manière analogue à celle décrite à l'exemple 11, mais à partir de 2,2 g d'acide bromo-3 benzoïque de 1,8 g de N,N'-carbonyldiimidazole et de 2,4 g d'amino-2 (bromo-3 phényl)-7 naphtyridine-1,8. Le produit obtenu par précipitation dans l'eau (3,1 g; F environ 140°C) est dissous dans 100 cm³ de propanol bouillant. Après 3 heures de refroidissement à 4°C le solide cristallisé est séparé par filtration, lavé par 2 fois 10 cm³ de propanol et séché à 40°C sous pression réduite (0,07 kPa). On obtient 2,2 g de N-[(bromo-3 phényl)-7 naphtyridine-1,8 yl-2] bromo-3 benzamide fondant à 144-146°C.

*Exemple 33*

On opère d'une manière analogue à celle décrite à l'exemple 11, mais à partir de 1,6 g d'acide bromo-3 benzoïque de 1,3 g de N,N'-carbonyldiimidazole et de 1,3 g d'amino-2 (méthoxy-2 phényl)-7 naphtyridine-1,8. Le produit obtenu par précipitation dans l'eau (1,5 g) est dissous dans 75 cm³ de méthanol bouillant. Après 2 heures de refroidissement à 4°C, le solide cristallisé est séparé par filtration, lavé par 3 fois 3 cm³ de méthanol et séché à 35°C sous pression réduite (0,07 kPa). On obtient 0,6 g de N-[(méthoxy-2 phényl)-7 naphtyridine-1,8 yl-2] bromo-3 benzamide fondant à 110°C.

L'amino-2 (méthoxy-2 phényl)-7 naphtyridine-1,8 peut être préparée de la façon suivante:

On opère d'une manière générale à celle décrite à l'exemple 11, mais à partir de 2,3 g de sodium, de 15 g de méthoxy-2 acétophénone et de 11 g de formiate d'éthyle. Les 19,8 g de solide obtenu sont alors ajoutés à 11,6 g de diamino-2,6 pyridine. Le produit obtenu après hydrolyse et neutralisation à la soude (10,9 g; F environ 145°C) est dissous dans 200 cm$^3$ de dichlorométhane et cristallisé par addition de 15 cm$^3$ d'acide chlorhydrique en solution 3N dans l'éther éthylique. Le produit cristallisé est séparé par filtration, lavé 3 fois 20 cm$^3$ de dichlorométhane puis 3 fois 10 cm$^3$ d'acétonitrile et dissous dans 60 cm$^3$ d'eau distillée. Le précipité obtenu après neutralisation par une solution aqueuse saturée de bicarbonate de sodium est séparé par filtration, lavé par 5 fois 15 cm$^3$ d'eau distillée et séché à l'air. On obtient 2,9 g d'amino-2 (méthoxy-2 phényl)-7 naphtyridine-1,8 fondant à 182°C.

*Exemple 34*

On opère d'une manière analogue à celle décrite à l'exemple 11, mais à partir de 4 g d'acide bromo-3 benzoïque, de 3,2 g de N,N'-carbonyldiimidazole et de 3,5 g d'amino-2 (méthoxy-3 phényl)-7 naphtyridine-1,8. Le produit obtenu par précipitation dans l'eau (4 g; F environ 90°C) est dissous dans 200 cm$^3$ d'acétonitrile bouillant. Après 3 heures de refroidissement à 4°C, le solide cristallisé est séparé par filtration, lavé par 3 fois 3 cm$^3$ d'acétonitrile et séché à 35°C sous pression réduite (0,07 kPa). On obtient 0,8 g de N-[(méthoxy-3 phényl)-7 naphtyridine-1,8 yl-2] bromo-3 benzamide fondant à 104°C.

L'amino-2 (méthoxy-3 phényl)-7 naphtyridine-1,8 peut être préparée de la façon suivante:

On opère d'une manière analogue à celle décrite à l'exemple 11, mais à partir de 2,3 g de sodium, de 15 g de méthoxy-3 acétophénone et de 11 g de formiate d'éthyle. Les 20 g de solide obtenus sont alors ajoutés à 11 g de diamino-2,6 pyridine. Le produit obtenu après hydrolyse et neutralisation par la soude est séparé par filtration, lavé par 6 fois 100 cm$^3$ d'eau distillée à 50°C, puis séché à l'air. On obtient 23,8 g d'amino-2 (méthoxy-3 phényl)-7 naphtyridine-1,8 fondant à 270-275°C.

*Exemple 35*

On opère d'une manière analogue à celle décrite à l'exemple 11, mais à partir de 2,8 g d'acide bromo-3 benzoïque de 2,3 g de N,N'-carbonyldiimidazole et de 2,4 g d'amino-2 (méthyl-3 phényl)-7 naphtyridine-1,8. Le produit obtenu par précipitation dans l'eau (2,3 g) est dissous dans 300 cm$^3$ d'acétonitrile bouillant. Après 4 heures de refroidissement à 4°C le solide cristallisé est séparé par filtration, lavé par 3 fois 5 cm$^3$ d'acétonitrile et séché à 35°C sous pression réduite (0,07 kPa). On obtient 1 g de N-[(méthyl-3 phényl)-7 naphtyridine-1,8 yl-2] bromo-3 benzamide fondant à 100°C.

L'amino-2 (méthyl-3 phényl)-7 naphtyridine-1,8 peut être préparée de la façon suivante:

On opère d'une manière analogue à celle décrite à l'exemple 11, mais à partir de 2,3 g de sodium, de 13,4 g de méthyl-3 acétophénone et de 11 g de formiate d'éthyle. Les 18,4 g de solide obtenu sont alors ajoutés à 12,1 g de diamino-2,6 pyridine. Le produit obtenu après hydrolyse et neutralisation à la soude (14 g; F environ 140°C) est dissous dans 100 cm$^3$ d'acide chlorhydrique concentré (d = 1,19). Le précipité obtenu après hydrolyse avec 500 cm$^3$ d'eau distillée puis neutraliation avec de la soude 10N, est séparé par filtration, lavé par 5 fois 50 cm$^3$ d'eau distillée et 2 fois 50 cm$^3$ de méthanol, puis séché à l'air. On obtient 7,3 g d'amino-2 (méthyl-3 phényl)-7 naphtyridine-1,8 fondant à 180°C.

*Exemple 36*

On opère d'une manière analogue à celle décrite à l'exemple 11, mais à partir de 2,1 g d'acide bromo-3 benzoïque de 1,6 g de N,N'-carbonyldiimidazole et de 2,1 g d'amino-2 (dichloro-3,4 phényl)-7 naphtyridine-1,8. Le produit obtenu par précipitation dans l'eau (2,5 g; F environ 150°C) est dissous dans un mélange de 75 cm$^3$ de diméthylfomamide et de 100 cm$^3$ d'acétonitrile bouillant. Après 4 heures de refroidissement à 4°C le solide cristallisé est séparé par filtration, lavé par 3 fois 5 cm$^3$ d'acétonitrile et séché à 40°C sous pression réduite (0,07 kPa). On obtient 1,2 g de N-[(dichloro-3,4 phényl)-7 naphtyridine-1,8 yl-2] bromo-3 benzamide fondant à 176-178°C.

La présente invention concerne également les compositions pharmaceutiques qui contiennent les produits de formule générale (I) à l'état pur ou associés à un adjuvant, un diluant et/ou un enrobage compatibles et phramaceutiquement acceptables. Ces compositions pharmaceutiques peuvent être employées par voie orale, rectale, parentérale, percutanée ou intranasale.

Comme compositions solides pour administration orale peuvent être utilisés des comprimés, des pilules, des poudres (généralement dans des caspules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent égalment comprendre des substances autres que les diluants, par exemple des produits mouillants, édulcorants ou aromatisants.

Les compositions selon l'invention pour administration intranasale ou parentérale peuvent être des solutions stériles aqueuses ou non aqueuses, des suspensions ou des émulsions. Comme solvant ou véhicule, on peut employer le propylèneglycol, un polyéthylèneglycol, des huiles végétales en particulier l'huile d'olive, d'amande ou de coco et des esters organiques injectables, par exemple l'oléate d'éthyle. Ces compositions peuvent également contenir des adjuvants, en particulier des agents mouillants, émulsifiants ou dispersants (lécithine de soja). La stérilisation peut se faire de plusieurs façons, par exemple en incorporant à la composition des agents stérilisants, par irradiation, par chauffage ou par

addition d'un agent conservateur. Elles peuvent être présentées sous forme de compositions solides stériles qui seront dissoutes au moment de l'emploi dans un milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine par leur action anti-infectieuse et immunorestauratrice.

En thérapeutique humaine, les doses dépendent de l'effet recherché et de la durée du traitement; elles sont généralement comprises entre 5 et 250 mg par jour par voie orale, ou comprises entre 0,5 et 25 mg par jour par voie parentérale pour un adulte.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent une composition selon l'invention.

*Exemple A*

On prépare selon la technique habituelle des comprimés dosés à 5 mg de produit actif ayant la composition suivante:

- N-(phényl-7 naphtyridine-1,8 yl-2) bromo-3 benzamide .................. 0,005 g
- amidon .................... 0,100 g
- silice précipitée ............... 0,018 g
- stéarate de magnésium ......... 0,002 g

*Exemple B*

On prépare selon la technique habituelle un soluté injectable ayant la composition suivante:

- N-(phényl-7 naphtyridine-1,8 yl-2) méthoxy-4 benzamide .................. 0,020 g
- propylèneglycol .................. 1 $cm^3$
- eau pour préparations injectables q.s.p . 2 $cm^3$

*Exemple C*

On prépare selon la technique habituelle des comprimés dosés à 5 mg de produit actif ayant la composition suivante:

- N-[(pyrrolidinyl-1)-7 naphtyridine-1,8 yl-2] benzamide .................. 0,005 g
- amidon .................... 0,100 g
- silice précipitée ............... 0,018 g
- stéarate de magnésium ......... 0,002 g

**Revendications pour les états contractants:**
BE, CH, DE, FR, GB, IT, LI, LU, NL, SE

1. Un nouveau dérivé d'amide substitué, caractérisé en ce qu'il répond à la formule générale:

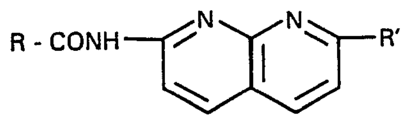

dans laquelle:

1) soit le symbole R représente un radical quinolyle-2, phényle substitué (par un atome de brome, par un atome de chlore en position -3, un radical trifluorométhyle ou alcoyloxycarbonyle, ou par un radical dialcoylamino en position -4) ou un radical dichlorophényle, trialcoyloxyphényle, benzyle, styryle, benzoyle ou anilino et le symbole R' représente un atome d'halogène, un radical phényle ou anilino, ou bien

le symbole R' représente un radical phénoxy ou chloro-4 phénoxy lorsque R est un radical quinolyle-2, phényle substitué (par un atome de brome ou en position -3 par un atome de chlore ou substitué par un radical trifluorométhyle ou alcoyloxycarbonyle ou en position -4 par un radical dialcoylamino), ou trialcoyloxyphényle, benzyle ou styryle, ou bien

le symbole R' représente un radical fluoro-4 phényle ou alcoyloxy-4 phényle lorsque R est un radical trifluorométhyl-4 phényle ou dialcoylamino-4 phényle, ou bien

le symbole R' représente un radical bromo-3 phényle lorsque R est un radical anilino,

2) soit le symbole R représente un radical phényle ou alcoyloxyphényle et

le symbole R' représente un radical phényle, chloro-4 phénoxy, anilino ou pyrrolidinyle-1, ou bien,

le symbole R' représente un radical chloro-2 (ou -4) phényle, bromo-3 (ou -4) phényle, alcoyloxy-4 phényle ou dichloro (ou dibromo)-3,4 phényle lorsque R est un radical phényle,

3) soit le symbole R représente un radical phényle substitué en position -3 par un atome d'halogène et le symbole R' représente un radical fluoro-3 (ou -4) phényle, brome-3 (ou -4) phényle, chlore-4 phényle, alcoyloxy-2 (ou -3) phényle, alcoyl-3 phényle ou difluoro (ou dichloro ou dibromo) -3, 4 phényle, étant entendu que les radicaux alcoyle cités ci-dessus sont droits ou ramifiés et contiennent 1 à 4 atomes de carbone.

2. Un nouveau dérivé d'amide selon la revendication 1, caractérisé en ce que

1) soit le symbole R représente un radical quinolyle-2, dialcoylamino-4 phényle, dichlorophényle, trialcoyloxyphényle, benzyle, styryle ou benzoyle et

le symbole R' représente un atome de chlore ou un radical phényle, ou bien

le symbole R' représente un radical phénoxy lorsque R est un radical bromophényle, ou bien

le symbole R représente un atome de chlore lorsque R est un radical bromophényle, trifluorométhylphényle ou anilino, ou bien

le symbole R' représente un radical bromo-3 phényle lorsque R est un radical anilino,

2) soit le symbole R représente un radical phényle ou alcoyloxyphényle et

le symbole R' représente un radical phényle, chloro-4 phénoxy, anilino ou pyrrolidinyle-1, ou bien, le symbole R' représente un radical chloro-2 (ou -4) phényle, bromo-3 (ou -4) phényle ou alcoyloxy-4 phényle lorsque R est un radical phényle,

3) soit le symbole R représente un radical phényle substitué en position -3 par un atome d'halogène et le symbole R' représente un radical fluoro-3 (ou -4) phényle, brome-3 (ou -4) phényle, chlore-4 phényle, alcoyloxy-2 (ou -3) phényle, alcoyl-3 phényle ou difluoro (ou dichloro ou dibromo) -3, 4 phényle, étant entendu que les radicaux alcoyle cités ci-dessus sont droits ou ramifiés et contiennent 1 à 4 atomes de carbone.

3. Le N-(phényl-7 naphtyridine-1,8 yl-2) bromo-3 benzamide.

4. Le N-(phényl-7 naphtyridine-1,8 yl-2) méthoxy-4 benzamide.

5. Le N-[(pyrrolidinyl-1)-7 naphtyridine-1,8 yl-2) benzamide.

6. Le N-[(chloro-4 phényl)-7 naphtyridine-1,8 yl-2) bromo-3 benzamide.

7. Le N-[(fluoro-4 phényl)-7 naphtyridine-1,8 yl-2) bromo-3 benzamide.

8. Un procédé de préparation d'un dérivé d'amide selon la revendication 1, pour lequel R est défini comme dans la revendication 1, à l'exception de représenter un radical anilino, et R' a la définition correspondante, caractérisé en ce que l'on fait agir un acide de formule générale:

R-COOH

dans laquelle R est défini comme ci-dessus, ou un dérivé réactif de cet acide sur une amine de formule générale:

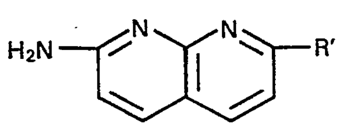

dans laquelle R' est défini comme dans la revendication 1.

9. Un procédé de préparation d'un dérivé d'amide selon la revendication 1, pour lequel R est un radical anilino et R' a la définition correspondante donnée dans la revendication 1, caractérisé en ce que l'on fait agir l'isocyanate de phényle sur une amine de formule générale:

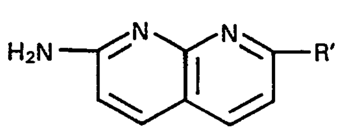

dans laquelle R' représente un atome d'halogène, ou un radical phényle, bromo-3 phényle ou anilino.

10. Composition pharmaceutique caractérisée en ce qu'elle contient au moins un produit selon la revendication 1 à l'état pur ou en association avec un ou plusieurs diluants ou adjuvants compatibles et pharmaceutiquement acceptables.

**Revendications pour l'état contractant:** AT

1. Un procédé de préparation d'un nouveau dérivé d'amide substitué, caractérisé en ce qu'il répond à la formule générale:

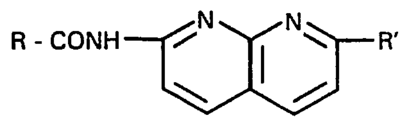

(I)

dans laquelle:

1) soit le symbole R représente un radical quinolyle-2, phényle substitué (par un atome de brome, par un atome de chlore en position -3, un radical trifluorométhyle ou alcoyloxycarbonyle, ou par un radical dialcoylamino en position -4) ou un radical dichlorophényle, trialcoyloxyphényle, benzyle, styryle, benzoyle ou anilino et le symbole R' représente un atome d'halogène, un radical phényle ou anilino, ou bien

le symbole R' représente un radical phénoxy ou chloro-4 phénoxy lorsque R est un radical quinolyle-2, phényle substitué (par un atome de brome ou en position -3 par un atome de chlore ou substitué par un radical trifluorométhyle ou alcoyloxycarbonyle ou en position -4 par un radical dialcoylamino), ou trialcoyloxyphényle, benzyle ou styryle, ou bien

le symbole R' représente un radical fluoro-4 phényle ou alcoyloxy-4 phényle lorsque R est un radical trifluorométhyl-4 phényle ou dialcoylamino-4 phényle, ou bien

le symbole R' représente un radical bromo-3 phényle lorsque R est un radical anilino,

2) soit le symbole R représente un radical phényle ou alcoyloxyphényle et

le symbole R' représente un radical phényle, chloro-4 phénoxy, anilino ou pyrrolidinyle-1, ou bien, le symbole R' représente un radical chloro-2 (ou -4) phényle, bromo-3 (ou -4) phényle, alcoyloxy-4 phényle ou dichloro (ou dibromo)-3,4 phényle lorsque R est un radical phényle,

3) soit le symbole R représente un radical phényle substitué en position -3 par un atome d'halogène et le symbole R' représente un radical fluoro-3 (ou -4) phényle, brome-3 (ou -4) phényle, chlore-4 phényle, alcoyloxy-2 (ou -3) phényle, alcoyl-3 phényle ou difluoro (ou dichloro ou dibromo) -3, 4 phényle, étant entendu que les radicaux alcoyle cités ci-dessus sont droits ou ramifiés et contiennent 1 à 4 atomes de carbone, caractérisé en ce que, pour obtenir un produit de formule générale (I) dans laquelle R est autre qu'anilino, on fait agir un acide de formule générale:

R-COOH (II)

dans laquelle R est défini comme ci-dessus, à l'exception de représenter un radical anilino ou un dérivé réactif de cet acide sur une amine de formule générale:

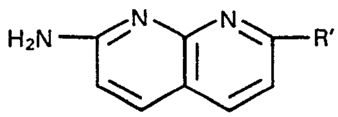

dans laquelle R' est défini comme ci-dessus, ou bien pour obtenir un produit de formule générale (I) dans laquelle R est un radical anilino, caractérisé en ce que l'on fait agir l'isocyante de phényle sur une amine de formule générale:

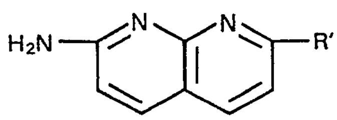

dans laquelle R' représente un atome d'halogène, ou un radical phényle, bromo-3 phényle ou anilino.

**Patentansprüche für die Vertragsstaaten:**
BE, CH, DE, FR, GB, IT, LI, LU, NL, SE

1. Ein neues substituiertes Amidderivat, dadurch gekennzeichnet, daß es der allgemeinen Formel:

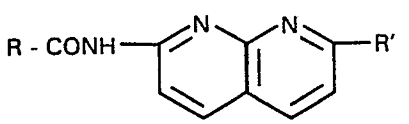

entspricht, worin:

1) entweder das Symbol R bedeutet einen Chinolyl-2-Rest, substituiertes Phenyl (durch ein Bromatom, durch ein Chloratom in 3-Stellung, einen Trifluormethyl- oder Alkyloxycarbonylrest, oder durch einen Dialkylaminorest in 4-Stellung) oder einen Dichlorphenyl-, Trialkyloxyphenyl-, Benzyl-, Styryl-, Benzoyl- oder Anilinorest und

das Symbol R' bedeutet ein Halogenatom, einen Phenyl- oder Anilinorest, oder

das Symbol R' bedeutet einen Phenoxy- oder 4-Chlorphenoxyrest, falls R ein Chinolyl-2-Rest, substituiertes Phenyl (durch ein Bromatom oder in 3-Stellung durch ein Chloratom oder substituiert durch einen Trifluormethyl- oder Alkoxycarbonylrest oder in 4-Stellung durch einen Dialkylaminorest), oder Trialkyloxyphenyl, Benzyl oder Styryl ist, oder das Symbol R' bedeutet einen 4-Fluorphenylrest oder 4-Alkyloxyphenylrest, falls R ein 4-Trifluormethylphenylrest oder 4-Dialkylaminophenylrest ist, oder

das Symbol R' bedeutet einen 3-Bromphenylrest, falls R ein Anilinorest ist,

2) oder das Symbol R bedeutet einen Phenyl- oder Alkoxyphenylrest und

das Symbol R' bedeutet einen Phenyl-, 4-Chlorphenoxy-, Anilino- oder Pyrrolidinyl-1-Rest, oder das Symbol R' bedeutet einen 2- (oder 4-) Chlorphenylrest, 3- (oder 4-) Bromphenylrest, 4-Alkoxyphenylrest oder 3, 4-Dichlor- (oder Dibrom-) phenylrest, falls R ein Phenylrest ist,

3) oder das Symbol R bedeutet einen Phenylrest, substituiert in 3-Stellung durch ein Halogenatom und

das Symbol R' bedeutet einen 3- (oder 4-) Fluorphenylrest, 3- (oder 4-) Bromphenylrest, 4-Chlorphenyl, 2- (oder 3-) Alkyloxyphenyl, 3-Alkylphenyl oder 3, 4-Difluor- (oder Dichlor- oder Dibrom-) phenyl,

wobei die vostehend erwähnten Alkylreste gerade oder verzweigt sind und 1 bis 4 Kohlenstoffatome enthalten.

2. Ein neues Amidderivat gemäß Anspruch 1, dadurch gekennzeichnet, daß

1) entweder das Symbol R bedeutet einen Rest Chinolyl-2, 4-Dialkylaminophenyl, Dichlorphenyl, Trialkoxyphenyl, Benzyl, Styryl oder Benzoyl und

das Symbol R' bedeutet ein Chloratom oder einen Phenylrest, oder

das Symbol R' bedeutet einen Phenoxyrest, falls R ein Bromphenylrest ist, oder

das Symbol R' bedeutet ein Chloratom, falls R ein Bromphenyl-, Trifluormethylphenyl- oder Anilinorest ist, oder

das Symbol R' bedeutet einen 3-Bromphenylrest, falls R ein Anilinorest ist

2) oder das Symbol R bedeutet einen Phenyl- oder Alkyloxyphenlrest und

das Symbol R' bedeutet einen Rest Phenyl, 4-Chlorphenoxy, Anilino oder Pyrrolidinyl-1, oder das Symbol R' bedeutet einen 2- (oder 4-) Chlorphenyl-, 3- (oder 4-) Bromphenyl- oder 4-Alkyloxyphenylrest, falls R ein Phenylrest ist,

3) oder das Symbol R bedeutet einen Phenylrest, substituiert in 3-Stellung durch ein Halogenatom und das Symbol R' bedeutet einen 3- (oder 4-) Fluorphenylrest, 3- (oder 4-) Bromphenylrest, 4-Chlorphenyl, 2- (oder 3-) Alkyloxyphenyl, 3-Alkylphenyl oder 3, 4-Difluor- (oder Dichlor- oder Dibrom-) phenyl,

wobei die vostehend erwähnten Alkylreste gerade oder verzweigt sind und 1 bis 4 Kohlenstoffatome enthalten.

3. N-(7-Penhyl-1,8-naphthyridin-2-yl)-3-brombenzamid.

4. N-(7-Phenyl-1,8-naphthyridin-2-yl)-4-methoxybenzamid.

5. N-[7-(Pyrrolidinyl-1)-1,8-naphthyridin-2-yl]-benzamid.

6. N-[7-(4-Chlorphenyl)-1,8-naphthyridin-2-yl]-3-bronzbenzamid.

7. N-[7-(4-Fluorphenyl)-1,8-naphthyridin-2-yl]-3-brombenzamid.

8. Verfahren zur Herstellung eines Amidderivats gemäß Anspruch 1, wobei R wie in Anspruch 1 definiert ist, mit Ausnahme der Bedeutung eines Anilinorestes, und R' die entsprechende Bedeutung hat, dadurch gekennzeichnet, daß man eine Säure der allgemeinen Formel:

R-COOH

worin R wie vorstehend definiert ist, oder in reaktionsfähiges Derivat dieser Säure auf ein Amin der allgemeinen Formel:

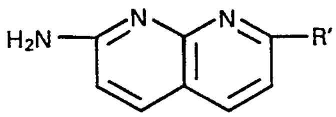

worin R' wie in Anspruch 1 definiert ist, einwirken läßt.

9. Verfahren zur Herstellung eines Amidderivats gemäß Anspruch 1, wobei R ein Anilinorest ist und R' die entsprechende, in Anspruch 1 angegebene Bedeutung hat, dadurch gekennzeichnet, daß man Phenylisocyanat auf ein Amin der allgemeinen Formel:

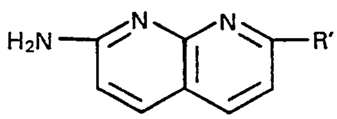

worin R' ein Halogenatom oder einen Phenyl-, 3-Bromphenyl- oder Anilinorest bedeutet, einwirken läßt.

10. Pharmazeutische Zusammensetzung, dadurch gekennzeichnet, daß sie mindestens ein Produkt gemäß Anspruch 1 in reinem Zustand oder zusammen mit einem oder mehreren verträglichen und pharmazeutisch annehmbaren Verdünnungs- oder Hilfsmitteln enthält.

**Patentanspruch für den Vertragsstaat:** AT

1. Verfahren zur Herstellung eines neuen substituierten Amidderivats, dadurch gekennzeichnet, daß es der allgemeinen Formel:

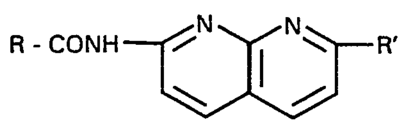 (I)

entspricht, in welcher:

1) das Symbol R einen 2-Chinolyl-, einen (durch ein Bromaton, durch ein Chloratom in 3-Stellung, einen Trifluormethyl- oder Alkyloxycarbonylrest oder durch einen Dialkylaminorest in 4-Stellung) substituierten Phenyl- oder einen Dichlorphenyl-, Trialkyloxyphenyl-, Benzyl-, Styryl-, Benzoyl- oder Anilinorest darstellt, und das Symbol R' stellt ein Halogenatom, einen Phenyl- oder Anilinorest dar, oder

das Symbol R' stellt einen Phenoxy- oder 4-Chlorphenoxyrest dar, wenn R ein 2-Chinolyl-, ein (durch ein Bromaton oder ein in 3-Stellung befindliches Chloratom, einen Trifluormethyl- oder Alkyloxycarbonylrest oder durch einen Dialkylaminorest in 4-Stellung) substituierter Phenylrest oder ein Trialkyloxyphenyl-, Benzyl- oder Styrylrest ist, oder

das Symbol R' stellt einen 4-Fluorphenyl- oder 4-Alkyloxyphenylrest dar, wenn R ein 4-Trifluormethylphenyl- oder 4-Dialkylaminophenylrest ist, oder

das Symbol R' stellt einen 3-Bromphenylrest dar, wenn R ein Anilinorest ist,

2) oder das Symbol R stellt einen Phenyl- oder Alkyloxyphenylrest dar und

das Symbol R' stellt einen Phenyl-, 4-Chlorphenoxy-, Anilino- oder 1-Pyrrolidinylrest dar, oder

das Symbol R' stellt einen 2-(oder 4-) Chlorphenyl, 3-(oder 4-)-Bromphenyl, 4-Alkyloxyphenyl- oder 3, 4-Dichlor- (oder -Dibrom-) phenylrest dar, wenn R ein Phenylrest ist,

3) oder das Symbol R stellt einen in 3-Stellung durch ein Halogenatom substituierten Phenylrest dar und

das Symbol R' stellt einen 3-(oder 4-) Fluorphenyl-, 3-(oder 4-)-bromphenyl-, 4-Chlorphenyl-, 2-(oder 3-) Alkyloxyphenyl 3-Alkylphenyl- oder 3, 4-Difluor-(-Dichlor- oder -Dibrom) phenylrest dar, wobei

die oben genannten Alkylreste selbstverständlich geradkettig oder verzweigt sind und 1 bis 4 Kohlenstoffatome enthalten, dadurch gekennzeichnet, daß man zur Herstellung einer Verbindung der allgemeinen Formel (I), in welcher R von Anilino verschieden ist, eine Säure der allgemeinen Formel:

R-COOH (II)

in welcher R die obige Bedeutung mit Ausnahme eines Anilinorestes hat, oder ein reaktionsfähiges Derivat dieser Säure auf ein Amin der allgemeinen Formel:

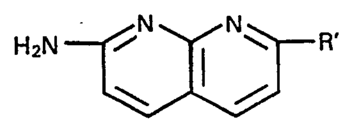

in welcher R' die obige Bedeutung hat, einwirken läßt, oder daß man zur Herstellung einer Verbindung der allgemeinen Formel (I), worin R ein Anilinorest ist, Phenylisocyanat mit einem Amin der allgemeinen Formel

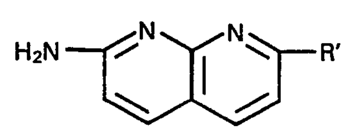

in welcher R' ein Halogenatom, einen Phenyl-, 3-Bromphenyl- oder Anilinorest darstellt, reagieren läßt.

**Claims for the Contracting States:**
BE, CH, DE, FR, GB, IT, LI, LU, NL, SE

1. New substituted amide derivative, characterized in that it corresponds to the general formula:

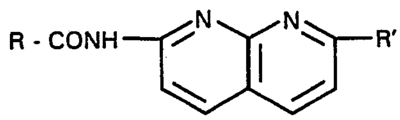

in which:

1) either the symbol R represents a 2-quinolyl radical, a substituted phenyl radical (substituted with a bromine atom, with a chlorine atom in position -3, a trifluoromethyl or alkyloxycarbonyl radical or with a dialkylamino radical in position -4) or a di-chlorophenyl, trialkyloxyphenyl, benzyl, styryl, benzoyl or anilino radical, and the symbol R′ represents a halogen atom, a phenyl or anilino radical, or

the symbol R′ represents a phenoxy or 4-chlorophenoxy radical when R is a 2-quinolyl radical, a substituted phenyl radical (substituted with a bromine atom or, in position -3, with a chlorine atom or substituted with a trifluormethyl or alkyloxycarbonyl radical or, in position -4, with a dialkylamino radical) or a trialkyloxyphenyl, benzyl or styryl radical, or

the symbol R′ represents a 4-fluorophenyl or 4-alkyloxyphenyl radical when R is a 4-trifluoromethylphenyl or 4-dialkylaminophenyl radical, or

the symbol R′ represents a 3-bromophenyl radical when R is an anilino radical,

2) or the symbol R represents a phenyl or alkyloxyphenyl radical and,

the symbol R′ represents a phenyl, 4-chlorophenoxy, anilino or 1-pyrrolidinyl radical, or the symbol R′ represents a 2-(or 4-) chlorophenyl, 3-(or 4-) bromophenyl, 4-alkyloxyphenyl or 3, 4-dichloro (or di-bromo) phenyl radical when R is a phenyl radical,

3) or the symbol R represents a phenyl radical substituted with a halogen atom in position -3, and the symbol R′ represents a 3-(or 4-) fluorophenyl, 3-(or 4-) bromophenyl, 4-chlorophenyl, 2-(or 3-) alkyloxyphenyl, 3-alkyphenyl, or 3, 4-difluoro (or dichloro or dibromo)- phenyl radical,

it being understood that the alkyl radicals mentioned above are straight-chain or branched and contain 1 to 4 carbon atoms.

2. New amide derivative according to Claim 1, characterized in that:

1) either the symbol R represents a 2-quinolyl, 4-dialkylaminophenyl, dichlorophenyl, trialkyloxyphenyl, benzyl, styryl or benzoyl radical and

the symbol R′ represents a chlorine atom or a phenyl radical, or

the symbol R′ represents a phenoxy radical when R′ is a bromophenyl radical, or

the symbol R′ represents a chlorine atom when R is a bromophenyl, trifluoromethylphenyl or anilino radical, or

the symbol R′ represents a 3-bromophenyl radical when R is an anilino radical,

2) or the symbol R represents a phenyl or alkyloxyphenyl radical and

the symbol R′ represents a phenyl, 4-chlorophenoxy, anilino or 1-pyrrolidinyl radical, or the symbol R′ represents a 2-(or 4-) chlorophenyl, 3-(or 4-) bromophenyl, or 4-alkyloxyphenyl radical when R is a phenyl radical,

3) or the symbol R represents a phenyl radical substituted in 3-position with a halogen atom and the symbol R′ represents a 3-(or 4-) fluorophenyl, 3-(or 4-) bromophenyl, 4-chlorophenyl, 2-(or 3-) alkyloxyphenyl, 3-alkyphenyl or 3, 4-difluoro (or dichloro or dibromo) phenyl radical, it being understood that the radicals mentioned above are straight-chain or branched and contain 1 to 4 carbon atoms.

3. N-(7-Phenyl-1,8-naphthyridin-2-yl)-3-bromobenzamide.

4. N-(7-Phenyl-1,8-naphthyridin-2-yl)-4-methoxybenzamide.

5. N-[7-(1-Pyrrolidinyl)-1,8-naphthyridin-2-yl]-benzamide.

6. N-[7-(4-Chlorophenyl)-1,8-naphthyridin-2-yl]-3-bromobenzamide.

7. N-[7-(4-Fluorophenyl)-1,8-naphthyridin-2-yl]-3-bromobenzamide.

8. Process for the preparation of an amide derivative according to Claim 1, in which R is defined as in Claim 1, with the exception of representing an anilino radical, and R′ has the corresponding definition, characterized in that an acid of general formula:

R-COOH

in which R is defined as above, or a reactive derivative of this acid, is reacted with an amine of general formula:

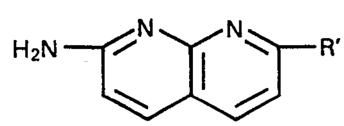

in which R′ is defined as in Claim 1.

9. Process for the preparation of an amide derivative according to Claim 1, in which R is an anilino radical and R′ has the corresponding definition given in Claim 1, characterized in that the phenyl isocyanate is reacted with an amine of general formula:

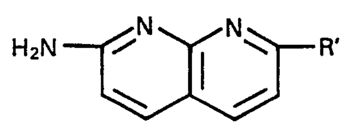

in which R′ represents a halogen atom, or a phenyl, 3-bromophenyl or anilino radical.

10. Pharmaceutical composition characterized in that it contains at least one product according to Claim 1 in the pure state or in combination with one or more compatible and pharmaceutically acceptable diluents or adjuvants.

**Claims for the Contracting State:** AT

1. Process for the preparation of a new substituted amide derivative, characterized in that it corresponds to the general formula:

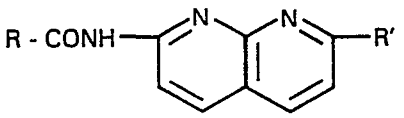

(I)

in which:

1) either the symbol R represents a 2-quinolyl radical, a substituted phenyl radical (subtituted with a bromine atom, with a chlorine atom in position -3, a trifluoromethyl or alkyloxycarbonyl radical or with a dialkylamino radical in position -4) or a di-chlorophenyl, trialkyloxyphenyl, benzyl, styryl, benzoyl or anilino radical, and the symbol R' represents a halogen atom, a phenyl or anilino radical, or

the symbol R' represents a phenoxy or 4-chlorophenoxy radical when R is a 2-quinolyl radical, a substituted phenyl radical (substitued with a bromine atom or, in position -3, with a chlorine atom or substitued with a trifluoromethyl or alkyloxycarbonyl radical or, in position -4, with a dialkylamino radical) or a trialkyloxyphenyl, benzyl or styryl radical, or

the symbol R' represents a 4-fluorophenyl or 4-alkyloxyphenyl radical when R is a 4-trifluoromethylphenyl or 4-dialkylaminophenyl radical, or

the symbol R' represents a 3-bromophenyl radical when R is an anilino radical,

2) or the symbol R represents a phenyl or alkyloxyphenyl radical, and

the symbol R' represents a phenyl, 4-chlorophenoxy, anilino or 1-pyrrolidinyl radical, or the symbol R' represents a 2-(or 4-) chlorophenyl, 3-(or 4-) bromophenyl, 4-alkyloxyphenyl or 3, 4-dichloro (or dibromo) phenyl radical when R is a phenyl radical,

3) or the symbol R represents a phenyl radical substituted with a halogen atom in position -3, and

the symbol R' represents a 3-(or 4-) fluorophenyl, 3-(or 4-) bromophenyl, 4-chlorophenyl, 2-(or 3-) alkyloxyphenyl, 3-alkylphenyl, or 3, 4-difluoro (or dichloro or dibromo)- phenyl radical,

it being understood that the alkyl radicals mentioned above are straight-chain or branched and contain 1 to 4 carbon atoms,

characterized in that, to obtain a product of general formula (I), in which R is other than anilino, an acid of general formula:

R-COOH (II)

in which R is defined as above with the exception that it does not represent an anilino radical, or a reactive derivative of this acid, is reacted with an amine of general formula:

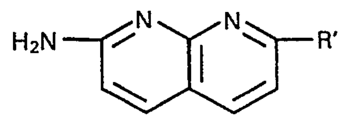

in which R' is defined as above, or to obtain a product of general formula (I), in which R is an anilino radical, characterized in that the phenyl isocyanate is reacted with an amine of general formula:

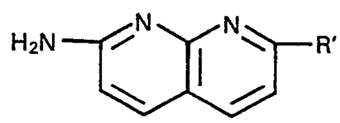

in which R' represents a halogen atom, or a phenyl, 3-bromophenyl or anilino radical.